Europäisches Patentamt

European Patent Office

Office européen des brevets

(19)

(11) Veröffentlichungsnummer: **0 299 160 B1**

(12) **EUROPÄISCHE PATENTSCHRIFT**

(45) Veröffentlichungstag der Patentschrift: **19.11.92**

(21) Anmeldenummer: **88107018.9**

(22) Anmeldetag: **02.05.88**

(51) Int. Cl.⁵: **A61B 17/58**

(54) Mit einem Knochen mittels einer schräg verlaufenden Schraube zu verbindende Platte.

(30) Priorität: **13.07.87 DE 8709615 U**

(43) Veröffentlichungstag der Anmeldung:
**18.01.89 Patentblatt 89/03**

(45) Bekanntmachung des Hinweises auf die
Patenterteilung:
**19.11.92 Patentblatt 92/47**

(84) Benannte Vertragsstaaten:
**DE ES FR GB IT**

(56) Entgegenhaltungen:
**EP-A- 0 217 317**
**DE-U- 8 124 272**
**LU-A- 85 888**

(73) Patentinhaber: **Waldemar Link GmbH & Co**
**Barkhausenweg 10**
**W-2000 Hamburg 63(DE)**

(72) Erfinder: **Keller, Arnold**
**An der Naherfurth 5**
**W-2061 Kayhude(DE)**

(74) Vertreter: **Glawe, Delfs, Moll & Partner Patent-**
**anwälte**
**Rothenbaumchaussee 58 Postfach 2570**
**W-2000 Hamburg 13(DE)**

Rank Xerox (UK) Business Services

## Beschreibung

Die Erfindung bezieht sich auf eine *Klingenplatte für Keilosteotomie nach dem Oberbegriff des Anspruchs 1.*

Bei *Klingenplatten für die supratuberculäre Keilosteotomie (DE-U 81 24 272)* will man nicht nur eine normal zur Knochenoberfläche verlaufende Anpreßkraft zwischen der Platte und dem Knochen, sondern auch eine Schrägzugkraft erzeugen, die dazu dient, die Osteotomiefläche des proximalen Knochenfragments gegen diejenige des distalen zu pressen. Dabei ist es bekannt, Sitz, Durchgangsloch und Ausfräsung des Schraubenlochs gleichachsig unter etwa 45° anzuordnen. Jedoch hat dies den Nachteil, daß der Winkelbereich innerhalb dessen die Schräglage der Schraube frei gewählt werden kann, stark begrenzt ist. Er umfaßt bei weitem nicht den mindestens erwünschten Bereich von 30° bis 60° gegenüber der Normalrichtung. - Bei den in der Praxis verwendeten Keilosteotomieplatten wird das Schraubenloch daher als Langloch ausgeführt, wobei die Sitzflächen parallel zur Plattenrichtung verlaufen; das heißt, daß ihre Achsrichtung normal zur Richtung der Platte und der Knochenoberfläche verläuft. Zwar ergibt dies einen wesentlich größeren Wahlwinkelbereich für die Schraube; jedoch bleibt die maximal verwendbare Schrägung begrenzt auf einen Wert weit unter 60°. Abhilfe würde eine Schrägstellung der Platte bringen, die aber aus anatomischen Gründen nicht in Frage kommt, oder eine Schrägstellung der das Langloch bildenden Flächen gegenüber der Platte, die aber an der begrenzten Dicke der Platte scheitert.

Der Erfindung liegt daher die Aufgabe zugrunde, eine Platte der eingangs genannten Art zu schaffen, deren Schraubenloch einen großen Winkelbereich für die Schrägstellung der Schraube und einen großen Schrägungswinkel gegenüber der Normalrichtung erlaubt.

Die erfindungsgemäße Lösung besteht darin, daß der Sitz schräg entsprechend einem mittleren Wert des vorbestimmten Winkelbereichs angeordnet ist, wobei das Zentrum des Sitzes, dessen Sitzdurchmesser größer als die Dicke der Platte ist, innerhalb der Plattenbegrenzung liegt, und daß das Durchgangsloch und die Ausfräsung unter einem größeren Winkel als der Sitz gegenüber der Normalrichtung geneigt sind.

Obwohl unter den bekannten Ausführungen lediglich das Langloch einen hinreichend großen Winkelbereich verspricht, geht die Erfindung demnach von dem nicht länglich gestreckten, sondern einfach gebohrten Schraubenloch aus. Den Nachteil der Beschränkung des Winkelbereichs sowie der Erreichbarkeit eines großen Schrägungswinkels sichert sie dadurch, daß der Sitz einerseits und das Durchgangsloch und die Ausfräsung andererseits eine stärker gegenüber der Normalrichtung geneigte Achsrichtung haben. Dadurch wird der erreichbare Schrägungswinkel gegenüber der Achsrichtung des Sitzes beträchtlich vergrößert, und zwar nicht nur in Richtung größerer Abweichung von der Normalrichtung, weil für den Schraubendurchgang bei geringerer Schrägung infolge der begrenzten Plattendicke ohnehin genügend freier Durchgangsquerschnitt zur Verfügung steht. Erforderlichenfalls werden das Durchgangsloch und die Ausfräsung zusätzlich zu der Richtung größerer Schrägung auch noch in geringerer Schrägungsrichtung erweitert.

Das Zentrum des Sitzes liegt zweckmäßigerweise ungefähr in der Mitte der Plattendicke, weil dann die Erzeugende der Sitzfläche sowohl auf der Außen- als auch auf der Innenseite über die Begrenzungsebenen der Platten hindurchtritt und damit Freiräume für den Schraubendurchgang schafft. Außerdem wird dadurch bewirkt, daß die Sitzflächen sich hauptsächlich seitlich derjenigen Ebene befindet, in der sich die Schraubenachse bei unterschiedlichen Schrägungen überwiegend befindet, während die in dieser Ebene liegenden Sitzflächenbereiche, die die Schwenkung der Schraube in dieser Ebene behindern könnten, wegfallen.

Zweckmäßigerweise ist diese Achse unter etwa 35° bis 55°, vorzugsweise etwa 45° gegenüber der Normalrichtung geneigt. Das Durchgangsloch und die Ausschrägung sind zweckmäßigerweise unter etwa 50° bis 70° gegenüber der Normalrichtung geneigt. Dies führt stets dann zu vernünftigen Ergebnissen, wenn der Durchmesser des Durchgangslochs mindestens etwa ebenso groß ist wie die Dicke der Platte, vorzugsweise mindestens etwa 1,5 mal so groß.

Die Schneide der Klingenplatte ist nach einem weiteren Merkmal durch eine beidseitig etwa gleiche Schrägung gebildet. Im Querschnitt erhält die Scheide dadurch eine symmetrische Gestalt und läßt sich dadurch richtungstreu einschlagen, während die bekannten Klingenplatten, bei denen die Schneide einerseits durch eine ebene, in Klingenrichtung verlaufende Fläche und andererseits durch einen schrägen Anschliff gebildet ist, leicht von der vorgesehenen Einschlagrichtung abweichen.

Die Erfindung wird im folgenden näher unter Bezugnahme auf die Zeichnung erläutert, die ein vorteilhaftes Ausführungsbeispiel veranschaulicht. Darin zeigen:

Fig. 1   eine Ansicht in Richtung des Pfeils I der Fig. 2,

Fig. 2   eine Ansicht in Richtung des Pfeils II der Fig. 1,

Fig. 3   eine Ansicht in Richtung des Pfeils III der Fig. 2,

Fig. 4 eine teilweise geschnittene Ansicht in Richtung des Pfeils IV der Fig. 2 und

Fig. 5 eine der Fig. 4 entsprechende Schnittansicht mit Knochenschraube.

Dargestellt ist eine Klingenplatte nach Giebel zur Kompressionsosteosynthese bei supratuberculärer Keilosteotomie. Selbstverständlich ist die Erfindung aber auch bei anderen Platten anwendbar.

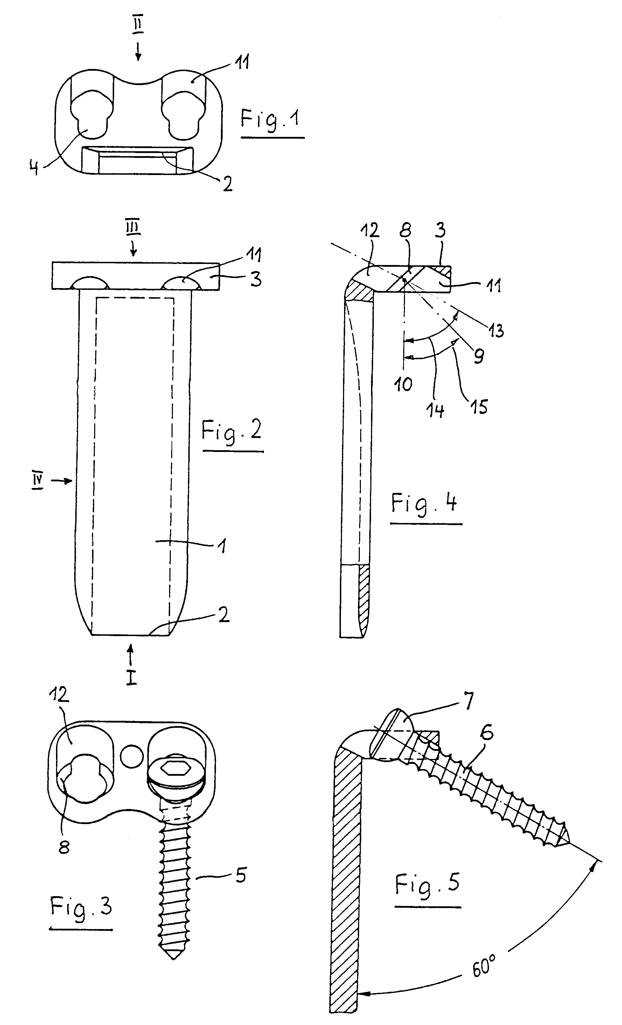

Die Klingenplatte besteht aus der Klinge 1, die in der Schneide 2 ausläuft, und der Kopfplatte 3, die winklig einstückig mit der Klinge 1 verbunden ist. Wie man in Fig. 4 erkennt, ist die Schneide 2 symmetrisch ausgebildet, das heißt, daß ihre Schneidrichtung mit der Längsrichtung der Klinge 1 übereinstimmt.

Die Kopfplatte 3 enthält zwei Schraubenlöcher 4 zur Aufnahme von Knochenschrauben 5, die einen Schaft 6 und einen vorzugsweise sphärisch unterseitig begrenzten Kopf 7 aufweisen. Jedes Schraubloch bildet etwa mittig zur Dicke der Kopfplatte 3 einen Sitz 8, der als Konus mit vorzugsweise 90° Spitzenwinkel ausgebildet ist aber auch eine andere Gestalt haben kann. Die Richtung der Achse 9 des Sitzes 8 verläuft unter einem Winkel 15 gegenüber der Normalrichtung 10 von etwa 45°. Zentrisch bezogen auf den Mittelpunkt des Sitzes 8, der etwa in der Mitte der Dicke der Kopfplatte 3 liegt, schließen sich innenseitig von dem Sitz 8 das Durchgangsloch 11 für den Schaft 6 der Schraube und außenseitig die Ausfräsung 12 für den Kopf 7 der Schraube an, deren Achsen 13 unter einem Winkel 14 von 60° bis 70° gegenüber der Normalrichtung 10 verlaufen. Die Ausfräsung 12 schneidet den Sitz auf der Außenseite (Ansicht Fig. 3) vollständig frei. Desgleichen ergibt sich durch die Durchgangsbohrung 11 auf der Innenseite (Ansicht Fig. 1) ein vollständiger Freischnitt, so daß der Schaft und der Kopf 7 der Schraube 5 innerhalb der Schwenkebene, die durch die Achsen 9, 10 und 13 festgelegt ist, nahezu jede beliebige Stellung innerhalb eines Winkels zwischen etwa 0 und 70° zur Normalrichtung stehen, einnehmen können, wobei die Unterfläche des Kopfes 7 stets einwandfrei mit dem Sitz 8 zusammenwirken kann. Dem Operateur wird dadurch ein wesentlich größeres Maß an Freiheit gewährt.

Wie man der Zeichnung entnimmt, ergeben sich die genannten Freischnittverhältnisse jeweils dann, wenn die Plattendicke in einem bestimmten Verhältnis zur Lage und zu den Durchmessern der Bohrungen liegt. Sehr vorteilhafte Verhältnisse ergeben sich bei einer Plattendicke von 4 mm, einem Durchmesser des Durchgangslochs von 6 mm, einem Durchmesser der zylindrischen Ausfräsung 12 von 8 bis 9 mm, einem Winkel 14 von 30° für die Achse 13 des Durchgangslochs 11 und 25° für die Ausfräsung 12 sowie einem Winkel 15 für die Achse 9 des Sitzes von 45°.

## Patentansprüche

1. Klingenplatte mit Klingenschneide (2) für Keilosteotomie, die mittels einer Schraube (5) mit dem Knochen zu verbinden ist, deren Achsrichtung (9) innerhalb eines vorbestimmten Winkelbereichs zwischen etwa 0° und 70° zur Normalrichtung (10) steht, und deren Schraubenloch einen Sitz (8) für den Schraubenkopf (7), ein engeres Durchgangsloch (11) für den Schraubenschaft (6) innenseitig von dem Sitz (8) und eine außenseitige Ausfräsung (12) umfaßt, dadurch gekennzeichnet, daß der Sitz (8) schräg entsprechend einem mittleren Wert des vorbestimmten Winkelbereichs angeordnet ist, wobei das Zentrum des Sitzes (8), dessen Sitzdurchmesser größer als die Dicke der Platte ist, innerhalb der Plattenbegrenzung liegt, und daß das Durchgangsloch (11) und die Ausfräsung (12) unter einem größeren Winkel als der Sitz (8) gegenüber der Normalrichtung (10) geneigt sind.

2. Platte nach Anspruch 1, dadurch gekennzeichnet, daß das Zentrum des Sitzes (8) ungefähr in der Mitte der Plattendicke liegt.

3. Platte nach Anspruch 1 oder 2, dadurch gekennzeichnet, daß die Sitzachse (9) unter etwa 35° bis 55° und die Achsen (13) des Durchgangslochs (11) und der Ausfräsung (12) unter etwa 50° bis 70° gegenüber der Normalrichtung (10) geneigt sind.

4. Platte nach einem der Ansprüche 1 bis 3, dadurch gekennzeichnet, daß die Klingenschneide (2) durch eine beidseitig etwa gleiche Schrägung gebildet ist.

## Claims

1. Plate blade with blade cutter (2) for cuneiform osteotomy, which is to be connected to the bone via a screw (5), the axial direction (9) of which is within a pre-set angle range of between about 0° and 70° to the normal direction (10), and the screw-hole of which comprises a seat (8) for the head of the screw (7), a narrower through hole (11) for the shaft of the screw (6) situated on the inside of the seat (8) and a countersink (12) situated on the outside of the seat (8), characterised in that the seat (8) is located obliquely corresponding to an average value of the pre-set angle range, the centre of the seat (8), the seat diameter of which is greater than the thickness of the plate, lying within the plate boundary, and in that the through hole (11) and the countersink (12) are inclined at a greater angle than the seat (8) from the normal direction (10).

2. Plate in accordance with Claim 1, characterised in that the centre of the seat (8) is approximately in the middle of the plate thickness.

3. Plate in accordance with Claim 1 or 2, characterised in that the axis of the seat (9) is inclined at approximately 35° to 55° and the axes (13) of the through hole (11) and the countersink (12) are inclined at approximately 50° to 70° from the normal direction (10).

4. Plate in accordance with one of Claims 1 to 3, characterised in that the blade cutter (2) is formed by a slant which is approximately equal on both sides.

**Revendications**

1. Plaque-lame à tranchant de lame (2) pour ostéotomie cunéiforme, destinée à être fixée à l'os au moyen d'une vis (5) dont la direction de l'axe (9) est située dans les limites d'une plage angulaire prédéterminée, comprise entre 0° et 70° par rapport à la direction normale (10), et dont le trou de vis comprend un siège (8) pour la tête de vis (7), un trou de passage (11) plus étroit pour la tige de vis (6) du côté interne du siège (8) et une fraisure (12) du côté externe, caractérisée en ce que le siège (8) est disposé avec une obliquité correspondant à une valeur moyenne de la plage angulaire prédéterminée, le centre du siège (8), dont le diamètre est plus grand que l'épaisseur de la plaque, étant situé à l'intérieur des limites de la plaque, et en ce que le trou de passage (11) et la fraisure (12) sont inclinés sous un angle plus grand que le siège (8) par rapport à la direction normale (10).

2. Plaque selon la revendication 1, caractérisée en ce que le centre du siège (8) est situé approximativement au milieu de l'épaisseur de la plaque.

3. Plaque selon la revendication 1 ou 2, caractérisée en ce que l'axe (9) du siège est incliné sous un angle d'environ 35° à 55° et les axes (13) du trou de passage (11) et de la fraisure (12) sont inclinés sous un angle d'environ 50° à 70° par rapport à la direction normale (10).

4. Plaque selon l'une quelconque des revendications 1 à 3, caractérisée en ce que le tranchant de lame (2) est formé par une inclinaison à peu près égale des deux côtés.

Fig.1

Fig.2

Fig.3

Fig.4

Fig.5